# EUROPEAN PATENT APPLICATION

(11) **EP 3 460 714 A1**
(43) Date of publication of application: **27.03.2019**
(21) Application number: 17897206.3
(22) Date of filing: 17.08.2017
(51) Int. Cl.: G06K 9/00

(54) **METHOD AND DEVICE FOR DETERMINING EVENT PERIOD VALUE**

(30) Priority: 08.08.2017 CN 201710669625
(71) Applicant: Wangsu Science & Technology Co., Ltd., Shanghai 200030 (CN)
(72) Inventor: CHEN, Xun, Shanghai 200030 (CN)
(74) Representative: Hanna Moore + Curley
(86) International application number: PCT/CN2017/097772
(87) International publication number: WO 2019/028931

(57) **Abstract**

The present disclosure provides a method and a device for determining an event periodic value. The method includes: acquiring a time series of a target event, where the time series includes a preset number of sequential values and the sequential values are configured to characterize the number of times that the target event occurs within unit time; calculating an autocorrelation sequence of the time series, and based on peak values and trough values of the autocorrelation sequence, determining a first candidate periodic value set corresponding to the autocorrelation sequence, where the candidate periodic values in the first candidate periodic value set are associated with confidence; calculating a Fourier transform result of the time series, and based on amplitude values of frequency points of the Fourier transform result, determining a second candidate periodic value set corresponding to the Fourier transform result, where candidate periodic values in the second candidate periodic value set are associated with confidence; acquiring a union of the first candidate periodic value set and the second candidate periodic value set, determining a total value of confidences corresponding to each candidate periodic value in the union, and based on the total value of confidences, determining the periodic value of the target event. Technical solutions of the present disclosure improve a determination precision of the event periodic value when the event periodic value can be automatically determined.

## Description

### FIELD OF THE DISCLOSURE

The present disclosure relates to the technical field of data processing and, more particularly, relates to a method and a system for determining an event periodic value.

### BACKGROUND

In natural society, many events occur following certain cycles. For example, the seasons in some regions change alternatingly and periodically between Spring, Summer, Fall, and Winter. For another example, the acoustic wave is the result of an object performing periodic vibration. Further, in humanistic society, in many industries that people work in, events also often occur following certain cycles. In one example, the bank may pay interests to clients on their saving accounts periodically. In another example, the employers pay the employees for their work periodically.

In actual lives, periodic events often occur along with similar types of random events, and as the noise, the random events often impact the recognition of periodicity of the events. Therefore, an effective algorithm is needed to correctly recognize the periodicity of the event under situations where noise exists.

Currently, determination of the periodic value of the occurring of an event is often realized by a periodogram method based on Fourier transform or by an autocorrelation algorithm. In particular, the periodogram method based on Fourier transform first performs Fourier transform on the processed time series, and then calculates square of the Fourier coefficient in the transform result, where the maximum value of the result corresponds to the periodic value of the original time series. The drawback of this method lies in the fact that: the detection accuracy of long-span periods is insufficient.

The autocorrelation algorithm determines the periodicity by evaluating the cross-correlation between the time series and the time series itself at different moments. The distance between the most significant group of peak values in the algorithm result indicates the periodicity of the original time series. The limitation of the autocorrelation algorithm lies in that: because there may be more than one group of peak values corresponding to the same period, it is relatively difficult to use the computer to automatically determine the important peak values from the output result of the algorithm. Thus, the user often needs to manually determine the most important group of peak values using the result image.

As such, a method that automatically determines the event periodic value with high precision is needed.

### BRIEF SUMMARY OF THE DISCLOSURE

Objective of the present disclosure is to provide a method and a device for determining an event periodic value, which improves determination precision of the event periodic value under situations where the event periodic value is automatically determined.

To realize the aforementioned objective, one aspect of the present disclosure provides a method for determining an event periodic value, and the method includes: acquiring a time series of a target event, where the time series includes a preset number of sequential values and the sequential values are configured to characterize the number of times that the target event occurs within unit time; calculating an autocorrelation sequence of the time series, and based on peak values and trough values of the autocorrelation sequence, determining a first candidate periodic value set corresponding to the autocorrelation sequence, where the candidate periodic values in the first candidate periodic value set are associated with confidence; calculating a Fourier transform result of the time series, and based on amplitude values of frequency points of the Fourier transform result, determining a second candidate periodic value set corresponding to the Fourier transform result, where candidate periodic values in the second candidate periodic value set are associated with confidence; determining an union of the first candidate periodic value set and the second candidate periodic value set to find a total value of confidences corresponding to each candidate periodic value in the union, and based on the total value of confidences, determining the periodic value of the target event.

Further, determining the first candidate periodic value set corresponding to the autocorrelation sequence includes: calculating a peak height corresponding to each peak value of the autocorrelation sequence, and based on the peak heights, extracting labeled peak values from the peak values of the autocorrelation sequence; calculating a time span between any two adjacent labeled peak values, and counting the repeated number of times that each time span occurs; using a time span whose number of repeated number of times satisfies designated conditions as a candidate periodic value of the autocorrelation sequence, and adding the candidate periodic value to the first candidate periodic value set.

Further, calculating a peak height corresponding to each peak value of the autocorrelation sequence includes: for each target peak value of the autocorrelation sequence, calculating a first difference and a second difference between the target peak value and its two adjacent trough values, respectively, and determining a smaller value from the first difference and the second difference as the peak height of the target peak value.

Further, extracting labeled peak values from the peak values of the autocorrelation sequence includes: comparing peak height corresponding to each peak value with a designated peak height threshold, and determining the peak values corresponding to peak height greater than or equal to the designated peak height threshold as the labeled peak values.

Further, the designated peak height threshold belongs to a threshold set, where the threshold set includes at least two different designated peak height thresholds. In particular, each designated peak height threshold corresponds to respectively extracted labeled peak values.

Further, the repeated number of times satisfies designated conditions include: the ratio of the repeated number of times to the total number of calculated time spans being greater than or equal to a designated ratio threshold.

Further, the following approach is applied to determine the confidence associated with a candidate periodic value in the first candidate periodic value set: using the ratio of the repeated number of times of the candidate periodic value to the total number of the calculated time spans as the confidence associated with the candidate periodic value.

Further, determining the second candidate periodic value set corresponding to the Fourier transform result includes: randomizing an ordering sequence of sequential values in the time series N times to obtain a number N of disordered time series, and calculating disordered Fourier transform results corresponding to the disordered time series, where N is an integer greater than or equal to 1; determining a maximal amplitude value from amplitude value of each frequency point in the disordered Fourier transform results; determining target frequency points with an amplitude value greater than or equal to the maximal amplitude value from the Fourier transform result of the time series, and using the reciprocals of the target frequency points as candidate periodic values in the second candidate periodic value set.

Further, the following approach is applied to determine the confidence associated with a candidate periodic value in the second candidate periodic value set: using the ratio of an amplitude value of a target frequency point to the maximal amplitude value as the confidence associated with the candidate periodic value corresponding to the target frequency point.

Further, determining the periodic value of the target event includes: determining one or more candidate periodic values with the total value of confidence being greater than or equal to the designated threshold to form the periodic value set of the target event.

Further, the method also includes: if the union is void, determining that the target event shows no periodicity.

To realize the aforementioned objective, another aspect of the present disclosure further provides a device for determining an event periodic value. The device includes a processor and a memory. The memory stores computer programs, and when the computer programs are executed by the processor, the following steps are implemented: acquiring a time series of a target event, where the time series includes a preset number of sequential values and the sequential values are configured to characterize the number of times that the target event occurs within unit time; calculating an autocorrelation sequence of the time series, and based on peak values and trough values of the autocorrelation sequence, determining a first candidate periodic value set corresponding to the autocorrelation sequence, where candidate periodic values in the first candidate periodic value set are associated with confidence; calculating a Fourier transform result of the time series, and based on amplitude values of frequency points of the Fourier transform result, determining a second candidate periodic value set corresponding to the Fourier transform result, where candidate periodic values in the second candidate periodic value set are associated with confidence; acquiring a union of the first candidate periodic value set and the second candidate periodic value set, determining a total value of confidences corresponding to each candidate periodic value in the union, and based on the total value of confidences, determining the periodic value of the target event.

Further, when the computer programs are executed by the processor, the following steps are also implemented: calculating a peak height corresponding to each peak value of the autocorrelation sequence, and based on the peak heights, extracting labeled peak values from the peak values of the autocorrelation sequence; calculating a time span between any two adjacent labeled peak values, and counting the repeated number of times that each time span occurs; using a time span whose repeated number of times satisfies designated conditions as a candidate periodic value of the autocorrelation sequence, and adding the candidate periodic value to the first candidate periodic value set.

Further, when the computer programs are executed by the processor, the following steps are also implemented: randomizing an ordering sequence of sequential values in the time series N times to obtain a number N of disordered time series, and calculating disordered Fourier transform results corresponding to the disordered time series, where N is an integer greater than or equal to 1; determining a maximal amplitude value from amplitude value of each frequency point in the disordered Fourier transform results; determining target frequency points with an amplitude value greater than or equal to the maximal amplitude value from the Fourier transform result of the time series, and using the reciprocals of the target frequency points as candidate periodic values in the second candidate periodic value set.

As such, based on combination of the autocorrelation algorithm and the Fourier algorithm, the present disclosure determines the first candidate periodic value set corresponding to the autocorrelation sequence through the peak values and trough values of the autocorrelation sequence. Further, through the amplitude values of the frequency points in the Fourier transform result, the second candidate periodic value set corresponding to the Fourier transform result may be determined. In the two candidate periodic sets, each candidate periodic value may be respectively included, and each candidate periodic value may be associated with the confidence. Thus, when finally determining the periodic value of the event, the union of the two sets may be determined. In the union, the total value of confidence of each candidate periodic value may be calculated. The total value of confidence may be the sum of the confidence of the same candidate periodic value in the two candidate periodic sets. Therefore, the periodic value of the target event may be determined based on the total value of the confidence. As such, through the combination of the two algorithms, the obtained precision of the periodic value may be higher, and the periodic value of the target event may be automatically determined by judgement through the total value of the confidence.

### BRIEF DESCRIPTION OF THE DRAWINGS

To more clearly illustrate technical solutions of the present disclosure, accompanying drawings used in descriptions of embodiments hereinbelow are introduced briefly. Obviously, the accompanying drawings described hereinafter are only some embodiments of the present disclosure, and for those ordinarily skilled in the relevant art, other drawings may be obtained from these accompanying drawings without creative labor.
FIG. 1 illustrates a method flow chart for determining an event periodic value according to Embodiment 1 of the present disclosure;
FIG. 2 is a schematic view illustrating determination of a threshold set according to Embodiment 1 of the present disclosure;
FIG. 3 is a diagram showing functional modules of a device according to Embodiment 2 of the present disclosure.

### DETAILED DESCRIPTION

To make the objective, technical solutions and advantages of the present disclosure clearer, embodiments of the present disclosure are described in more details with reference to the accompanying drawings.

### Embodiment 1

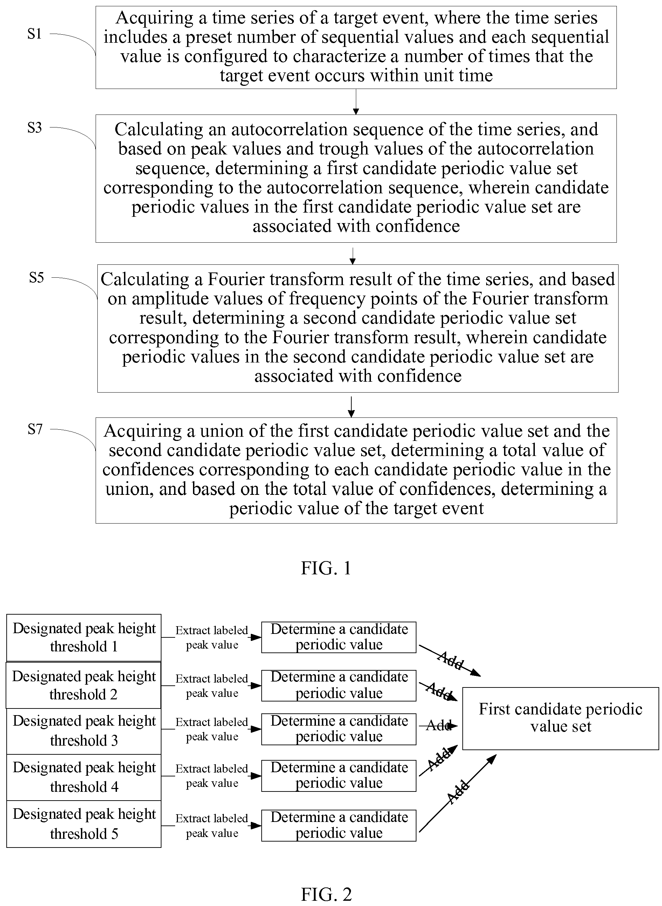

The present disclosure provides a method for determining an event periodic value. Referring to FIG. 1, the method includes following steps.

S1: acquiring a time series of a target event, where the time series includes a preset number of sequential values and the sequential values are configured to characterize the number of times that the target event occurs within unit time.

In some embodiments, the number of times that a to-be-analyzed target event occurs within unit time is counted. The unit time may be flexibly adjusted based on actual application scenarios. For example, the unit time may be approximately 1 hour, such that the number of times that the target event occurs within each hour can be counted.

In some embodiments, after the number of times that the target event occurs within the unit time is counted, the time series of the target event may be generated. The time series may include a preset number of sequential values, the preset number of sequential values are configured to characterize the number of times that the target event occurs within unit time, and every sequential value may correspond to the unit time. For example, the time series of the target event may be {2, 3, 6, 8, 9}, and each digit in the time series represents the number of times that the target event occurs within the unit time. If the unit time is approximately 1 hour, the counting period to obtain each digit in the time series is approximately 1 hour.

S3: calculating an autocorrelation sequence of the time series, and based on peak values and trough values of the autocorrelation sequence, determining a first candidate periodic value set corresponding to the autocorrelation sequence, where candidate periodic values in the first candidate periodic value set are associated with confidence.

In some embodiments, directed towards the time series, the corresponding autocorrelation sequence may be obtained by calculation through the autocorrelation algorithm. The equation to calculate the autocorrelation sequence is a technical approach commonly used in the relevant field, which is not repeatedly described herein. The autocorrelation sequence may be represented as {R*ᵢ*}, where the value of *i* ranges from 1 to m, and m is the total number of numeric values in the autocorrelation sequence.

In some embodiments, the numeric values in the autocorrelation sequence may be in a fluctuating state. That is, the numeric values in the autocorrelation sequence may correspond to peak values and trough values. The peak values and the trough values may be extracted from the autocorrelation sequence, where a peak value is greater than its two adjacent numeric values, and a trough value is smaller than its two adjacent numeric values.

After the peak values and the trough values are extracted, the peak height corresponding to each peak value in the autocorrelation sequence may be calculated. In some embodiments, the peak height may be determined using the following approach: for a target peak value of the autocorrelation sequence, calculating a first difference and a second difference between the target peak value and its two adjacent trough values, respectively, and determining a smaller value from the first difference and the second difference as the peak height of the target peak value. For example, the peak value may be 12, and the two adjacent trough values may be 3 and 7, respectively. Thus, the first difference and the second difference may be 9 and 5, respectively, and 5 may be determined as the peak height of the peak value 12.

In some embodiments, after determining the peak height of each peak value, the labeled peak value may be extracted from the peak values of the autocorrelation sequence based on the peak height. Specifically, the peak height corresponding to each peak value may be compared with the designated peak height threshold, and when the peak height is greater than or equal to the designated peak height threshold, the corresponding peak values are determined as the labeled peak values. For example, the designated peak height threshold may be 5, and the peak values with peak height greater than or equal to 5 are treated as the labeled peak values.

In some embodiments, after the labeled peak values are determined, the time span between two adjacent labeled peak values is then a periodic value corresponding to the time series. Therefore, the time span between any two adjacent labeled peak values may be calculated, and the repeated number of times that each time span occurs may be counted. In particular, the greater the repeated number of times, the higher the possibility that the corresponding time span may be used as the periodic value of the time series. For example, there may be 11 labeled peak values in total, and the time span between any two adjacent labeled peak values may be: 3 hours, 4 hours, 3 hours, 3 hours, 3 hours, 4 hours, 3 hours, 6 hours, 4 hours, and 3 hours, which yields 10 time spans. Further, the repeated number of times corresponding to the 3-hour time span is counted to be 6, the repeated number of times corresponding to the 4-hour time span is counted to be 3, and the repeated number of times for all other time spans is 1. In some embodiments, a time span whose repeated number of times satisfies designated conditions may be used as a candidate periodic value of the autocorrelation sequence, and the candidate periodic value may be added to the first candidate periodic value set. The designated conditions that the repeated number of times satisfies may include: the ratio of the repeated number of times to the total number of calculated time spans being greater than or equal to a designated ratio threshold. Assume the designated ratio threshold is 50%, according to the aforementioned example, the repeated number of times corresponding to the 3-hour time span accounts for a ratio of 60% of the total number, which exceeds the designated ratio threshold, while the ratios of the repeated number of times corresponding to other time spans with respect to the total number all do not exceed 50%. Thus, the 3-hour may be added to the first candidate periodic value set as a candidate periodic value.

In actual application scenarios, the number of candidate periodic values obtained through one designated peak height threshold may be relatively small, and to avoid leaving out periodic values possibly existing in the time series, a threshold set H may be provided in advance, and in the threshold set, there may be at least two designated peak height thresholds. Referring to FIG. 2, directed towards each designated peak height threshold, the labeled peak values may be obtained through the aforementioned approach. As such, each designated peak height threshold may correspond to the respectively extracted labeled peak values. Further, the candidate periodic values may be determined based on the labeled peak values extracted each time, such that the candidate periodic values in the first candidate periodic value set can be relatively complete. In some embodiments, the designated peak height thresholds in the threshold set may include a value-ranging interval. Based on the value-ranging interval and the fixed step length, each designated peak height threshold may be obtained. For example, the value-ranging interval corresponding to the threshold set may be [0, 6], and the fixed step length may be 2. Thus, three designated peak height thresholds 2, 4, and 6 may be generated.

In some embodiments, after each candidate periodic value in the first candidate periodic value set is determined, confidence associated with each candidate periodic value may be configured. The confidence may be configured to indicate the possibility of using the candidate periodic value as the practical periodic value. Specifically, the ratio of the repeated number of times of the candidate periodic value to the total number of the calculated time spans may be used as the confidence associated with the candidate periodic value. For example, in the above-described example, the confidence of the 3-hour candidate periodic value is 0.6.

S5: calculating a Fourier transform result of the time series, and based on amplitude values of frequency points of the Fourier transform result, determining a second candidate periodic value set corresponding to the Fourier transform result, where candidate periodic values in the second candidate periodic value set are associated with confidence.

In some embodiments, the second candidate periodic value set may be determined by the approach based on Fourier transform. Specifically, discrete Fourier transform (DFT) may be performed on the time series to obtain a Fourier transform result corresponding to the time series. In the Fourier transform result, the amplitude value corresponding to each frequency point may be displayed through an approach of periodogram. In particular, the greater the amplitude value, the higher the confidence that the frequency point corresponding to the amplitude value can be used as the frequency value of the time series (i.e., the reciprocal of the periodic value). Based on this, in some embodiments, to determine whether periodicity exists in the time series, the ordering sequence of sequential values in the time series may be randomized to obtain a disordered time series. If the original time series has certain periodicity, the disordered time series obtained after random rearrangement may show no significant periodicity. In practical application scenarios, to ensure the integrity of random rearrangement, the step of random rearrangement may be executed N times to obtain a number N of disordered time series, where N is an integer greater than or equal to 1.

In some embodiments, the DFT equation may be utilized to calculate the disordered Fourier transform result that respectively corresponds to each disordered time series, thereby obtaining a N number of disordered Fourier transform results. From the amplitude value of each frequency point from the N number of disordered Fourier transform results, the maximal amplitude value may be determined. The frequency point to which the maximal amplitude value corresponds may represent the periodic value possibly possessed by the disordered time series after the original time series is randomized. If the original time series possesses apparent periodicity, the amplitude value of the frequency point corresponding to the periodicity shall be greater than the maximal amplitude value obtained based on the disordered time series. Based on this, the target frequency points with amplitude values greater than or equal to the maximal amplitude value may be determined from the Fourier transform result of the time series, and the reciprocals of the target frequency points may be used as the candidate periodic values in the second candidate periodic value set. For example, the maximal amplitude value determined based on the disordered Fourier transform result may be 15. Correspondingly, the frequency points with amplitude value greater than or equal to 15 may be extracted from the Fourier transform result of the normal time series. Further, the reciprocal of each extracted frequency point may be calculated and used as a candidate periodic value in the second candidate periodic value set.

Similarly, after the candidate periodic values of the second candidate periodic value set are determined, confidence associated with each candidate periodic value may be configured. In some embodiment, the ratio of the amplitude value of a target frequency point to the maximal amplitude value may be used as the confidence associated with the candidate periodic value corresponding to the target frequency point. For example, the maximal amplitude value may be 15. If the amplitude value of a target frequency point is 30, the confidence associated with the candidate frequency value corresponding to the target frequency point may thus be 2.

S7: acquiring a union of the first candidate periodic value set and the second candidate periodic value set, determining a total value of confidences corresponding to each candidate periodic value in the union, and based on the total value of confidences, determining the periodic value of the target event.

In some embodiments, after the first candidate periodic value set and the second candidate periodic value set are determined, the union of the two sets may be determined, thereby combining the candidate periodic values from the two sets. After combination, some candidate periodic values may correspond to one confidence, and some candidate periodic values may correspond to two confidences. A candidate periodic value corresponding to two confidences indicates that the candidate periodic value simultaneously exists in the two sets, and the possibility of such candidate periodic value being the practical periodic value of the time series is relatively high. Thus, a total value of confidences corresponding to each candidate periodic value in the union may be determined, and based on the total value of confidences, the periodic value of the target event may be determined. Specifically, the total value of confidences may be a sum of the confidences from the two sets. When determining the periodic value of the target event, one or more candidate periodic values with the total value of confidences being greater than or equal to a designated threshold may be determined to form a periodic value set of the target event. It should be noted that, the number of determined periodic values may be more than one. Thus, it is indicated that in the original time series, two different periodic values may exist, which is relatively common in practical application scenarios.

Obviously, if the union is void, it is indicated that the first candidate periodic value set and the second candidate periodic value set are both voids. That is, no candidate periodic value exists, and it is determined that the target event has no periodicity.

### Embodiment 2

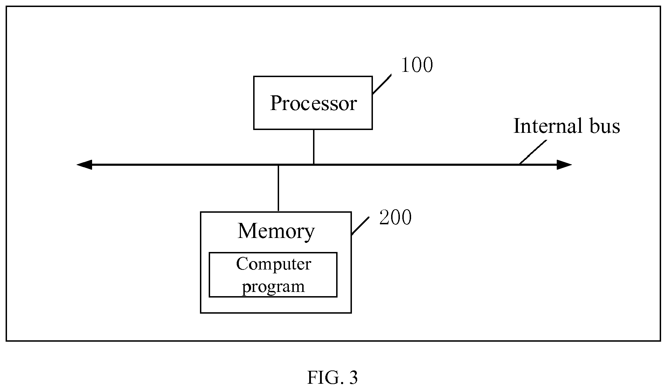

Referring to FIG. 3, the present disclosure further provides a device for determining an event periodic value. The device includes a processor 100 and a memory 200. The memory 200 stores computer programs, and when the computer programs are executed by the processor 100, the following steps are executed:

acquiring a time series of a target event, where the time series includes a preset number of sequential values and the sequential values are configured to characterize the number of times that the target event occurs within unit time;

calculating an autocorrelation sequence of the time series, and based on peak values and trough values of the autocorrelation sequence, determining a first candidate periodic value set corresponding to the autocorrelation sequence, where candidate periodic values in the first candidate periodic value set are associated with confidence;

calculating a Fourier transform result of the time series, and based on amplitude values of frequency points of the Fourier transform result, determining a second candidate periodic value set corresponding to the Fourier transform result, where candidate periodic values in the second candidate periodic value set are associated with confidence;

acquiring a union of the first candidate periodic value set and the second candidate periodic value set, determining a total value of confidences corresponding to each candidate periodic value in the union, and based on the total value of confidences, determining the periodic value of the target event.

In some embodiments, when the computer programs are executed by the processor, the following steps are realized:

calculating a peak height corresponding to each peak value of the autocorrelation sequence, and based on the peak heights, extracting labeled peak values from the peak values of the autocorrelation sequence;

calculating a time span between any two adjacent labeled peak values, and counting the repeated number of times that each time span occurs;

using a time span whose repeated number of times satisfies designated conditions as a candidate periodic value of the autocorrelation sequence, and adding the candidate periodic value to the first candidate periodic value set.

In some embodiments, when the computer programs are executed by the processor, the following steps are further implemented:

randomizing an ordering sequence of sequential values in the time series N times to obtain a number N of disordered time series, and calculating disordered Fourier transform results corresponding to the disordered time series, where N is an integer greater than or equal to 1;

determining a maximal amplitude value from amplitude value of each frequency point in the disordered Fourier transform results;

determining target frequency points with an amplitude value greater than or equal to the maximal amplitude value from the Fourier transform result of the time series, and using the reciprocals of the target frequency points as candidate periodic values in the second candidate periodic value set.

Each implementation of the present disclosure is described in a progressive manner, and the same or similar portions between different embodiments may refer to each other, and each implementation highlights the illustrations different from other implementations. In particular, directed towards the device embodiments, introduction of the aforementioned method embodiment may be applied for illustrations and explanations.

The present disclosure may be described in the general context of computer-executable instructions, such as program modules, being executed by a computer. Generally, program modules include routines, programs, objects, components, data structures, etc. that perform particular tasks or implement particular abstract data types. The invention may also be practiced in distributed computing environments where tasks are performed by remote processing devices that are linked through a communications network. In a distributed computing environment, program modules may be located in both local and remote computer storage media including memory storage devices.

From foregoing descriptions, the present disclosure combines the autocorrelation algorithm and the Fourier algorithm, and through the peak values and trough values of the autocorrelation sequence, the first candidate periodic value set corresponding to the autocorrelation sequence is determined. Further, through the amplitude values of the frequency points in the Fourier transform result, the second candidate periodic value set corresponding to the Fourier transform result may be determined. In the two candidate periodic sets, each candidate periodic value may be respectively included, and each candidate periodic value may be associated with the confidence. Thus, when finally determining the periodic value of the event, the union of the two sets may be determined. In the union, the total value of confidence of each candidate periodic value may be calculated. The total value of confidence may be the sum of the confidence of the same candidate periodic value in the two candidate periodic sets. Therefore, the periodic value of the target event may be determined based on the total value of the confidence. As such, through the combination of the two algorithms, the obtained precision of the periodic value may be higher, and the periodic value of the target event may be automatically determined by judgement through the total value of the confidence.

The sequence numbers of the embodiments of the present invention are merely for ease of description, and do not imply the preference throughout the embodiments.

The above-described device embodiments are for illustrative purposes, units described as separated components may or may not be physically separated, and the components displayed as units may or may not be physical units. Based on practical demands, partial or entire modules may be selected to implement the objective of the present disclosure. For those ordinarily skilled in the relevant art, the present disclosure may be understood and implemented without contributing creative labor.

Through the foregoing description of the implementation manners, it is clear to those skilled in the relevant art that the present disclosure may be implemented by software plus a necessary universal hardware platform, and certainly may also be implemented by hardware, but in many cases, the software implementation is preferred. Based on such an understanding, the technical solutions of the present disclosure or the part that makes contributions to the existing technology may be substantially embodied in the form of a software product. The software product may be stored in a computer-readable storage medium, such as ROM/RAM, magnetic disk, or optical disc, and includes a plurality of commands to instruct a computer device (which may be a personal computer, a server, or a network device) to perform the method according to each or some portions of embodiments of the present disclosure.

The foregoing is only preferred implementations of the present disclosure, which does not intend to limit the present disclosure. Without departing from the spirit of the present disclosure, any modification, equivalent replacement or improvement shall all fall within the protection scope of the present disclosure.

## Claims

1. A method for determining an event periodic value, **characterized in that**, the method comprising:
acquiring a time series of a target event, wherein the time series includes a preset number of sequential values and each sequential value is configured to characterize a number of times that the target event occurs within unit time;
calculating an autocorrelation sequence of the time series, and based on peak values and trough values of the autocorrelation sequence, determining a first candidate periodic value set corresponding to the autocorrelation sequence, wherein candidate periodic values in the first candidate periodic value set are associated with confidence;
calculating a Fourier transform result of the time series, and based on amplitude values of frequency points of the Fourier transform result, determining a second candidate periodic value set corresponding to the Fourier transform result, wherein candidate periodic values in the second candidate periodic value set are associated with confidence;
acquiring a union of the first candidate periodic value set and the second candidate periodic value set, determining a total value of confidences corresponding to each candidate periodic value in the union, and based on the total value of confidences, determining a periodic value of the target event.

2. The method according to claim 1, **characterized in that**, determining the first candidate periodic value set corresponding to the autocorrelation sequence comprises:
calculating a peak height corresponding to each peak value of the autocorrelation sequence, and based on the peak height, extracting labeled peak values from the peak values of the autocorrelation sequence;
calculating a time span between any two adjacent labeled peak values, and counting a repeated number of times that each time span occurs;
using a time span whose number of repeated number of times satisfies designated conditions as a candidate periodic value of the autocorrelation sequence, and adding the candidate periodic value to the first candidate periodic value set.

3. The method according to claim 2, **characterized in that**, calculating the peak height corresponding to each peak value of the autocorrelation sequence comprises:
for every target peak value of the autocorrelation sequence, calculating a first difference and a second difference between the target peak value and its two adjacent trough values, respectively, and determining a smaller value from the first difference and the second difference as a peak height of the target peak value.

4. The method according to claim 2 or 3, **characterized in that**, extracting the labeled peak values from the peak values of the autocorrelation sequence comprises:
comparing peak height corresponding to each peak value with a designated peak height threshold, and determining peak values corresponding to peak height greater than or equal to the designated peak height threshold as the labeled peak values.

5. The method according to claim 4, **characterized in that**, the designated peak height threshold belongs to a threshold set, the threshold set includes at least two different designated peak height thresholds, and each designated peak height threshold corresponds to respectively extracted labeled peak values.

6. The method according to claim 2, **characterized in that**, the repeated number of times satisfying designated conditions comprises:
a ratio of the repeated number of times to a total number of calculated time spans being greater than or equal to a designated ratio threshold.

7. The method according to claim 2, **characterized in that**, a following approach is applied to determine confidence associated with a candidate periodic value in the first candidate periodic value set:
using a ratio of the repeated number of times of the candidate periodic value to a total number of calculated time spans as the confidence associated with the candidate periodic value.

8. The device according to claim 1, **characterized in that**, determining the second candidate periodic value set corresponding to the Fourier transform result comprises:
randomizing an ordering sequence of sequential values in the time series N times to obtain a number N of disordered time series, and calculating disordered Fourier transform results corresponding to the disordered time series, wherein N is an integer greater than or equal to 1;
determining a maximal amplitude value from amplitude value of each frequency point in disordered Fourier transform results;
determining target frequency points with an amplitude value greater than or equal to the maximal amplitude value from the Fourier transform result of the time series, and using reciprocals of the target frequency points as candidate periodic values in the second candidate periodic value set.

9. The method according to claim 8, **characterized in that**, a following approach is applied to determine the confidence associated with the candidate periodic value in the second candidate periodic value set:
using a ratio of an amplitude value of a target frequency point to the maximal amplitude value as the confidence associated with the candidate periodic value corresponding to the target frequency point.

10. The method according to claim 1, **characterized in that**, determining the periodic value of the target event comprises:
determining one or more candidate periodic values with the total value of confidence being greater than or equal to a designated threshold to form a periodic value set of the target event.

11. The method according to claim 1, **characterized in that**, the method further comprising:
if the union is void, it is determined that the target event shows no periodicity.

12. A device for determining an event periodic value, **characterized in that**, the device includes a processor and a memory, the memory stores computer programs, and when the computer programs are executed by the processor, following steps are executed:
acquiring a time series of a target event, wherein the time series includes a preset number of sequential values and the sequential values are configured to characterize a number of times that the target event occurs within unit time;
calculating an autocorrelation sequence of the time series, and based on peak values and trough values of the autocorrelation sequence, determining a first candidate periodic value set corresponding to the autocorrelation sequence, wherein candidate periodic values in the first candidate periodic value set are associated with confidence;
calculating a Fourier transform result of the time series, and based on amplitude values of frequency points of the Fourier transform result, determining a second candidate periodic value set corresponding to the Fourier transform result, wherein candidate periodic values in the second candidate periodic value set are associated with confidence;
acquiring a union of the first candidate periodic value set and the second candidate periodic value set, determining a total value of confidences corresponding to each candidate periodic value in the union, and based on the total value of confidences, determining a periodic value of the target event.

13. The device according to claim 12, **characterized in that**, when the computer programs are executed by the processor, following steps are also executed:
calculating a peak height corresponding to each peak value of the autocorrelation sequence, and based on the peak height, extracting labeled peak values from the peak values of the autocorrelation sequence;
calculating a time span between any two adjacent labeled peak values, and counting a repeated number of times that each time span occurs;
using a time span whose number of repeated number of times satisfies designated conditions as a candidate periodic value of the autocorrelation sequence, and adding the candidate periodic value to the first candidate periodic value set.

14. The device according to claim 12, **characterized in that**, when the computer programs are executed by the processor, following steps are also executed:
randomizing an ordering sequence of sequential values in the time series N times to obtain a number N of disordered time series, and calculating disordered Fourier transform results corresponding to the disordered time series, wherein N is an integer greater than or equal to 1;
determining a maximal amplitude value from amplitude value of each frequency point in disordered Fourier transform results;
determining target frequency points with an amplitude value greater than or equal to the maximal amplitude value from the Fourier transform result of the time series, and using reciprocals of the target frequency points as candidate periodic values in the second candidate periodic value set.
